(19) Europäisches Patentamt
European Patent Office
Office européen des brevets

(11) Publication number : **0 502 668 A1**

(12) **EUROPEAN PATENT APPLICATION**

(21) Application number : **92301732.1**

(22) Date of filing : **28.02.92**

(51) Int. Cl.[5] : **C07D 471/06,** A61K 31/435, // (C07D471/06, 235:00, 221:00)

(30) Priority : **05.03.91 GB 9104548**

(43) Date of publication of application : **09.09.92 Bulletin 92/37**

(84) Designated Contracting States : **PT**

(71) Applicant : **BRITISH TECHNOLOGY GROUP LTD**
**101 Newington Causeway**
**London SE1 6BU (GB)**

(72) Inventor : **Cholody, Wieslaw Marek**
**Kusocinskiego Str. 1/17**
**84-200 Wejherowo (PL)**
Inventor : **Konopa, Jerzy Kazimierz**
**Bitwy Pod Lenino Str. 40**
**80-809 Gdansk (PL)**

(74) Representative : **Percy, Richard Keith et al**
**Patents Department British Technology Group Ltd 101 Newington Causeway**
**London SE1 6BU (GB)**

(54) **Imidazoacridines and their antineoplastic use.**

(57) Compounds of formula I

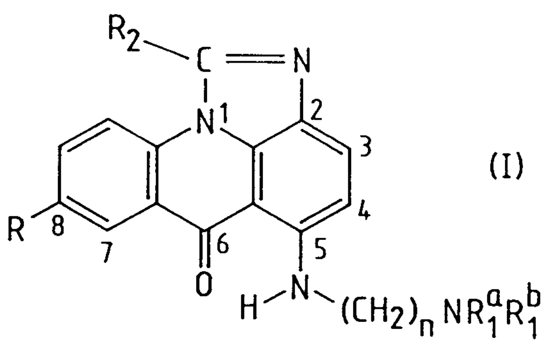

(I)

in which :

R represents : -OH or -OR′, wherein R′ represents $C_1$-$C_6$ alkyl,

$R_1^a$ and $R_1^b$, which may be identical or different, represent hydrogen or $C_1$-$C_6$ alkyl, unsubstituted or substituted by a hydroxyl, an amino, a N′-alkylamino or a N′,N′-dialkylamino group, such N′-alkyl groups containing 1-4 carbon atoms,

n is 2-5 and

$R_2$ represents hydrogen, or straight chain $C_{1-4}$ alkyl,

in the form of a free base or a pharmaceutically acceptable acid addition salt or an N-oxide are useful in antineoplastic treatment and prophylaxis, especially of leukaemias.

EP 0 502 668 A1

## Background of the Invention

### 1. Field of the invention

This invention relates to substituted 5-[(aminoalkyl)amino]-imidazo[4,5,1-de]acridin-6-ones and their antineoplastic use, especially for the treatment of leukaemia, and processes for their production.

### 2. Description of the related art

In the paper "5-[(aminoalkyl)amino]imidazo[4,5,1-de)acridin-6-ones as a novel class of antineoplastic agents. Synthesis and Biological Activity", J. Med. Chem. 33, 49-52 (1990), W.M. Cholody, S. Martelli, J. Paradziej-Lukowicz and J. Konopa have briefly reviewed tricyclic ring antineoplastic agents and have described new compounds recited in the title. A later paper, by W. Cholody et al., J. Med. Chem. 33, 2852-2856 (1990), entitled "8-substituted 5-[(aminoalkyl)aminol]-6H-v-triazolo[5,4,1-de]acridin-6-ones as potential antineoplastic agents. Synthesis and Biological Activity", describes these compounds, which differ by the replacement of an optionally substituted imidazo C-atom by a triazolo N-atom (unsubstituted) and optionally by the introduction of an 8-substituent selected from nitro, chloro, methyl, methoxy and hydroxy. The results are described as clearly indicating that high antineoplastic activity of those triazolo ring compounds is related to the A ring hydroxylation. However, structure-activity relationships in relation to *in vivo* evaluation against murine leukaemia remained unclear. It is, therefore, still a problem to find other compounds with high antineoplastic, especially antileukaemic, activity.

## Summary of the Invention

The present invention provides compounds of formula I:

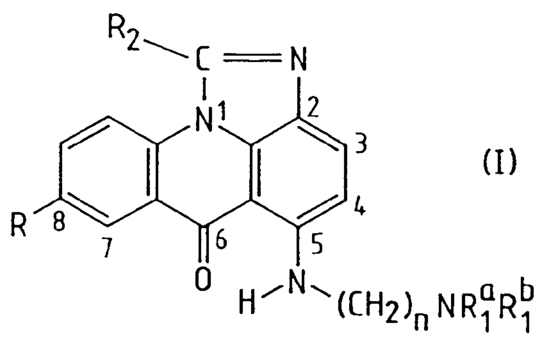

(I)

in which:

R represents: -OH or -OR′ wherein R′ represents $C_1$-$C_6$ alkyl, e.g. methyl,

$R_1^a$ and $R_1^b$, which may be identical or different, represent hydrogen or $C_1$-$C_6$ alkyl, e.g. methyl or ethyl, which is unsubstituted or is substituted by a hydroxyl, an amino, a N′-alkylamino or a N′,N′-dialkylamino group, in which the or each alkyl group has 1 - 4 carbon atoms, for example in the substituents: 2-hydroxyethyl, 2-aminoethyl, 2-(N′-alkylamino)ethyl and 2-(N′,N′-dialkylamino)ethyl, n is 2-5 and

$R_2$ represents hydrogen, or straight chain $C_{1-4}$ alkyl, in the form of the free bases, their pharmaceutically acceptable acid addition salts or N-oxides thereof.

## Description of the preferred embodiments

$R_1^a$ and $R_1^b$ are normally identical and represent alkyl groups e.g. $C_1$-$C_6$ alkyl groups, especially $C_1$-$C_3$ alkyl, such as methyl or ethyl, n typically being 2 or 3.

The compounds of formula I in which $R_1^a$ = $R_1^b$ = methyl or ethyl, $R_2$ = hydrogen or methyl and n is 2 or 3 are of particular interest in treating leukaemia. Of these compounds, those of most interest are those in which:

| | | | | |
|---|---|---|---|---|
| Ex. 9 | $R = -OH$; | $R_1{}^a = R_1{}^b = -CH_3$; | $R_2 = H$; | $n = 2$. |
| Ex. 10 | $R = -OH$; | $R_1{}^a = R_1{}^b = -CH_3$; | $R_2 = CH_3$; | $n = 2$. |
| Ex. 11 | $R = -OH$; | $R_1{}^a = R_1{}^b = -CH_2CH_3$; | $R_2 = H$; | $n = 2$. |
| Ex. 12 | $R = -OH$; | $R_1{}^a = R_1{}^b = -CH_2CH_3$; | $R_2 = CH_3$; | $n = 2$. |
| Ex. 14 | $R = -OH$; | $R_1{}^a = R_1{}^b = -CH_3$; | $R_2 = CH_3$; | $n = 3$. |
| Ex. 15 | $R = -OH$; | $R_1{}^a = R_1{}^b = -CH_2CH_3$; | $R_2 = H$; | $n = 3$. |
| Ex. 16 | $R = -OH$; | $R_1{}^a = R_1{}^b = -CH_2CH_3$; | $R_2 = CH_3$; | $n = 3$. |

The methyl ethers are generally less active, but among them the compounds in which $n = 2$, $R_2 = H$ and $R_1{}^a = R_1{}^b = CH_3$ or $C_2H_5$, (Examples 1 and 2) are the most preferred.

Addition salts which are generally pharmaceutically acceptable, may be of an organic or inorganic acid. Examples of suitable acids for salt formation are: hydrochloric, sulfuric, phosphoric, acetic, citric, malonic, ascorbic, maleic, methanesulfonic, lactic, gluconic, glucuronic, and the like. Usually the compound I is present in the form of a hydrochloride, which could be a mono-, di- or tri- salt or any mixture thereof. It can also be hydrated to variable extents.

It is known that N-oxides of chemotherapeutic anthraquinones having tertiary amino groups are useful as pro-drugs for antineoplastic therapy, being bioreductively activated within neoplastic tissue to form the active compound: see UK Patent Specification 2 237 283A (NRDC). Mild oxidation, under conditions which will not cause disruption of the imidazole ring, can be used to make N-oxides, of the imidazole N-atom or of a tertiary amine group present when $R_1{}^a$ and $R_1{}^b$ are not hydrogen atoms.

Compounds of formula I (in which $R_2$ represents hydrogen or a said alkyl group) may be produced as free bases or salts by treating a compound of formula II, optionally in the form of an acid addition salt thereof,

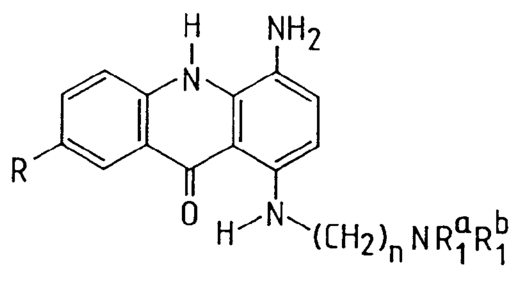

(II)

respectively with formic acid or a compound of formula $R_2CON(CH_3)_2$, preferably at elevated temperature, typically at reflux in the absence of added solvent. Salts can readily be converted to free bases and free bases to salts or N-oxides by methods known per se.

Compounds of formula II, optionally in the form of acid addition salts, may be produced from compounds of formula III by treatment thereof to reduce the nitro group to the corresponding amino group:

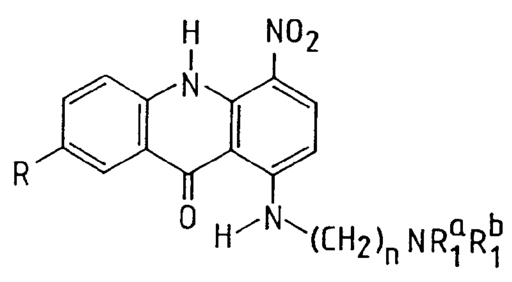

(III)

Compounds of formula III can be prepared as described in European Patent Application EP-A 145 226 (Warner-Lambert Company) or procedure: analogous thereto. The reduction of the nitro group is preferably carried out by hydrazine hydrate, suitably in the presence of a catalyst, e.g. Raney Nickel, in a polar solvent such as tetrahydrofuran (THF). The intermediates II thus obtained are generally extremely unstable to oxygen, especially in those compounds wherein n represents 3 and are usually used as starting materials for conversion to compounds of formula I in the form of acid addition salts, for example hydrochlorides.

The present invention further includes within its scope an intermediate of formula II, preferably in the form of an acid addition salt.

Two methods are generally used for isolation of the final products. In the case of methoxy derivatives, the products may be extracted with benzene or chloroform from the reaction mixture after rendering the mixture alkaline and next transformed into dihydrochlorides. The hydroxy compounds may instead be isolated as hydrochloride salts directly from the reaction mixture after acidification with HCl.

Compounds of formula I are of interest for the treatment or prophylaxis of neoplasms (a term used herein to refer to any malignant tissue growth and therefore to include cancers and leukaemias), especially lymphoblastic leukaemias. All the compounds of the invention tested appear to have antileukaemic activity in the in vitro and/or in vivo tests reported hereinafter (see Table III). In vivo tests of selected compounds of the invention against tumours in animals have shown that the compounds of Examples 11 and 12 are active against the MT-3 human mammary caninoma and the compounds of Examples 12 and 15 against colon 38 adenocarcinoma and B16 melanoma. In vitro data shows that compounds of Examples 10 and 16 have potent activity against renal cancer cell lines.

Accordingly, in a further aspect the present invention comprises a compound of formula I for use in therapy and, in a yet further aspect of the present invention, comprises the use of a compound of formula I for the manufacture of a medicament useful in antineoplastic treatment or prophylaxis.

The dosage form and amount can be readily established by reference to known antineoplastic treatment or prophylactic regimens. In general, however, the dosage of the compound of formula I usually lies within the range about 0.1mg to about 50mg/kg, preferably 0.5mg to 10mg/kg.

The compound of formula I can be administered alone or in connection with, one or more pharmaceutically acceptable diluents or carriers therefor, and optionally, any other ingredients which may be therapeutic per se, synergistic with the compound of formula I, or both. Carrier(s) and diluents are, of course, pharmaceutically acceptable and compatible with the other ingredients of the formulation.

Formulations suitable for oral, rectal, topical or parenteral (including subcutaneous, intramuscular and intravenous) administration are included. Unit dosage forms may be prepared by known methods. All methods generally include the step of bringing the active compound into association with a carrier or diluent, as a suspension or solution, and optionally one or more accessory ingredients, e.g. buffers, flavouring agents, binders, surface active agents, thickeners, anti-caking agents, lubricants and preservatives (including antioxidants).

Formulations of the present invention suitable for oral administration may be presented as capsules, cachets, tablets or lozenges, powder or granules; or a suspension. Tablets may be prepared by compressing the active compound in a free-flowing form such as a powder or granules, optionally mixed with conventional additives. A syrup may be made by adding the active compound to a concentrated, aqueous solution of a sugar, for example sucrose, optionally containing conventional additives. Formulations suitable for parenteral administration conveniently comprise a sterile aqueous preparation of the active compound which is preferably made isotonic.

The present invention is illustrated by the following Examples:-

## EXAMPLES

### General Procedure

Compounds of formula I, the subject of Examples 1 to 16, are produced by the route outlined in Scheme 1.

SCHEME 1

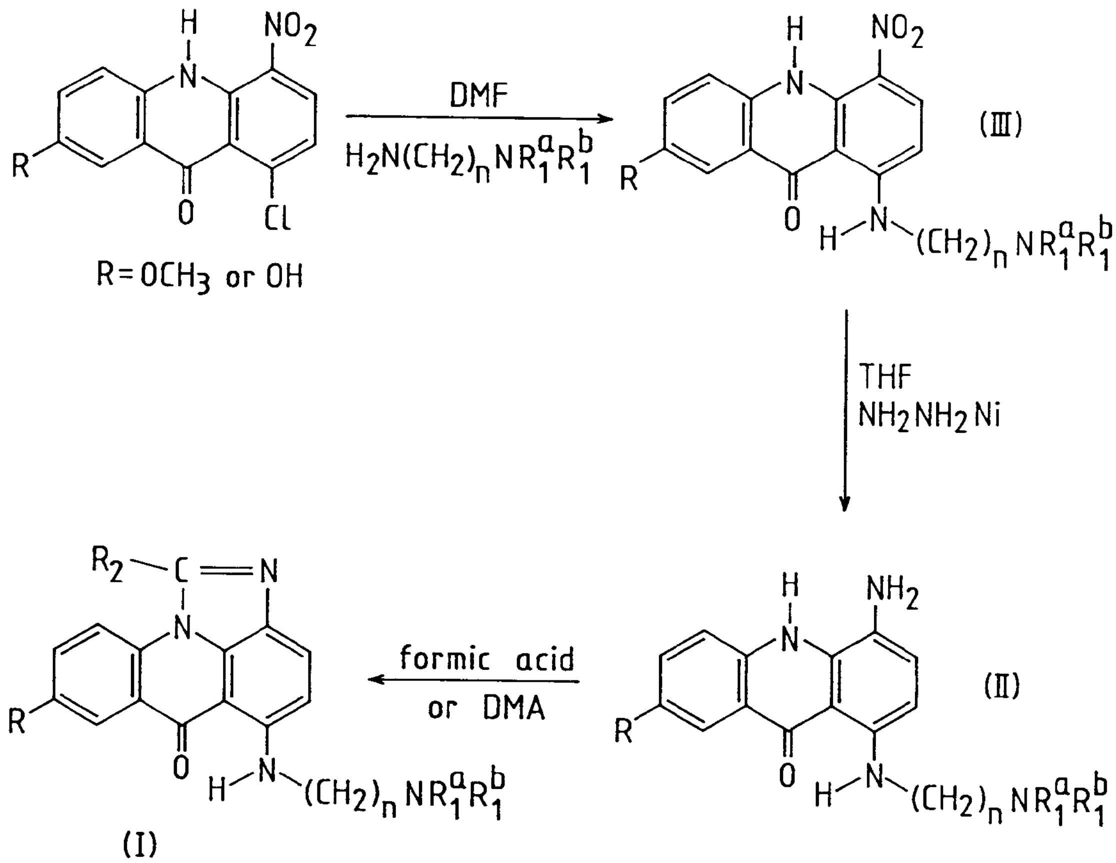


$R = OCH_3$ or OH

(III)

(II)

(I)

DMF = dimethylformamide,

THF = tetrahydrofuran,

DMA = dimethylacetamide.

In the following Examples melting points were taken on a Buchi 510 capillary melting points apparatus and are uncorrected. $^1H$ NMR spectra were recorded on a Varian VXR-300 spectrometer operating at 300 MHz. Chemical shifts are reported as $\delta$ units in ppm downfield from internal tetramethylsilane. NMR abbreviations used are as follows: br(broad), s(singlet), d(doublet), t(triplet), qu(quartet), qt(quintet), m(multiplet), ex(exchangeable with deuterium oxide). Quartets which by addition of deuterium oxide are transformed into triplets are labeled with *. Single frequency decoupling was utilized to assign specific protons. Coupling constants are given in Hz. Microanalytical results, indicated by atomic symbols, are within ±0.4% of the theoretical values.

EXAMPLE 1

A. 1-[[2-(Diethylamino)ethyl]amino]-7-methoxy-4-nitro-9(10H)-acridinone.

A mixture of 4.57g (0.015 mol) 1-chloro-7-methoxy-4-nitro-9(10H)-acridinone, 25 ml DMF and 7.00g (0.06

mol) 2-diethylaminoethylamine is stirred and heated at 60°C for 30 minutes. 100 ml 40% (v/v) MeOH-water solution is added to the reaction mixture, heated to boiling and after cooling left overnight in a refrigerator. The crystallized product is collected by filtration washed with water (150 ml) and MeOH (50 ml) and dried to give 5.30g. (92%) analytically pure product as yellow needles: mp 178-179°C (lit.mp., European Patent Appl. EP-A 145226, Chem. Abstr. 1985, 103, 215182s., 179-180°C);

B. Preparation of 7-substituted-4-amino-1-[[(dialkylamino)alkyl]amino]-9(10H)-acridinone Hydrochloride Salts.

To a mixture of nitro derivatives (0.01 mol), 200 ml THF, and about 2.5g of Raney Ni is added with stirring at room temperature then 2 ml hydrazine monohydrate. Stirring is continued for about 30 minutes. The catalyst is filtered off and washed with THF (50 ml). The filtrate is quickly treated with 10 ml concentrated hydrochloric acid and stirred for 10 minutes. The yellow precipitate obtained is collected and washed with THF. The product is recrystallized from a solution of MeOH (90%)dioxane made acidic with HCl (pH~2).

C. Preparation of 5-[[2-(diethylamino)ethyl]amino]-8-methoxyimidazo[4,5,1-de] acridin-6-one Dihydrochloride.

A mixture of 1.71g (4 mmol) of the product from the procedure of Example 1B and 20 ml 95% formic acid is heated at reflux for 6h. Acid is evaporated and the residue is dissolved in water (100 ml). The solution is made basic (pH 9) by addition of sodium carbonate and product is extracted with chloroform (2 x 100 ml). The organic extracts are dried and evaporated to give a residue which is dissolved in EtOH. The solution is made acidic with HCl and product is crystallized by addition of acetone to give the title product.

EXAMPLE 2

Compound I: n=2, R=$OCH_3$, $R_1^a$=$R_1^b$=$CH_3$, $R_2$=H.

The procedures 1A, 1B and 1C of Example 1 are followed but dimethylaminoethylamine is used in place of diethylaminoethylamine in 1A.

EXAMPLE 3

Compound I: n=2, R=$OCH_3$, $R_1^a$=$R_1^b$=$CH_3$, $R_2$=$CH_3$.

The procedures 1A and 1B of Example 1 are followed using dimethylaminoethylamine in place of diethylaminoethylamine. The product is then subjected to the following procedure (designated 3C):

A mixture of 2.14g (5 mmol) hydrochloride and 30 ml DMA is refluxed for 12h, 200 ml water is added to the reaction mixture, made basic with sodium hydroxide and the product is extracted with benzene (2.150 ml). The extracts are evaporated to dryness and the residue is dissolved in methanol-dioxane (1:1) mixture. The solution is made acidic with gaseous HCl and the crystallized product is collected by filtration to give yellow crystals.

EXAMPLE 4

Compound I: n=2, R=$OCH_3$, $R_1^a$=$R_1^b$=$CH_2CH_3$, $R_2$=$CH_3$.

The procedures 1A and 1B of Example 1 are followed and the product is subjected to procedure 3C.

EXAMPLE 5

Compound I: n=3, R=$OCH_3$, $R_1^a$=$R_1^b$=$CH_3$, $R_2$=H.

The procedures 1A, 1B and 1C of Example 1 are followed but dimethylaminopropylamine is used in place of diethylaminoethylamine in procedure 1A.

EXAMPLE 6

Compound I: n=3, R=$OCH_3$, $R_1\underline{a}$=$R_1\underline{b}$=$CH_3$, $R_2$=$CH_3$.

The procedure of Example 5 is followed but procedure 3C replaces procedure 1C.

EXAMPLE 7

Compound I: n=3, R=$OCH_3$, $R_1\underline{a}$=$R_1\underline{b}$=$CH_2CH_3$, $R_2$=H.

The procedures 1A, 1B and 1C of Example 1 are followed but diethylaminopropylamine is used in place of diethylaminoethylamine in procedure 1A.

EXAMPLE 8

Compound I: n=3, R=$OCH_3$, $R_1\underline{a}$=$R_1\underline{b}$=$CH_2CH_3$, $R_2$=$CH_3$.

The procedure of Example 7 is followed but procedure 3C replaces procedure 1C.

EXAMPLE 9

Compound I: n=2, R=OH, $R_1\underline{a}$=$R_1\underline{b}$=$CH_3$, $R_2$=H.

The procedure of Example 1 is followed but dimethylaminoethylamine is used in place of diethylaminoethylamine and 1-chloro-7-hydroxy-4-nitro-9(10H)-acridone is used in place of 1-chloro-7-methoxy-4-nitro-9(10H)-acridone in procedure 1A and procedure 1C is replaced by the following, designated 9C:

A mixture of 5 mmol of dihydrochloride salt and 20 ml of 95% formic acid is refluxed for 8h. Formic acid is evaporated and the residue is dissolved on heating in methanol. 3 ml conc. hydrochloric acid is added to the hot solution and the product is crystallized by addition of acetone. The product is collected by filtration and recrystallized from a methanol-acetone mixture.

EXAMPLE 10

Compound I: n=2, R=OH, $R_1\underline{a}$=$R_1\underline{b}$=$CH_3$, $R_2$=$CH_3$.

The procedure of Example 9 is followed but the following procedure, designated 10C, replaces 9C:

A mixture of 5 mmol of dihydrochloride salt and 25 ml of DMA is refluxed for 12h. About 20 ml of the solvent is evaporated, 100 ml acetone is added to the residue and the solution is acidified with gaseous HCl. The precipitated product is collected by filtration and washed with acetone. Crude product is recrystallized (if necessary twice) from methanol-acetone to give the respective dihydrochloride salt.

EXAMPLE 11

Compound I: n=2, R=OH, $R_1\underline{a}$=$R_1\underline{b}$=$CH_2CH_3$, $R_2$=H.

The procedure of Example 9 is followed but diethylaminoethylamine is used in place of dimethylaminoethylamine in procedure 1A.

EXAMPLE 12

Compound I: n=2, R=OH, $R_1\underline{a}$=$R_1\underline{b}$=$CH_2CH_3$, $R_2$=$CH_3$.

The procedure of Example 10 is followed but diethylaminoethylamine is used in place of dimethylaminoethylamine in procedure 1A.

EXAMPLE 13

Compound I: n=3, R=OH, $R_1\underline{a}=R_1\underline{b}=CH_3$, $R_2=H$.

The procedure of Example 9 is followed but dimethylaminopropylamine is used in place of dimethylaminoethylamine in procedure 1A.

EXAMPLE 14

Compound I: n=3, R=OH, $R_1\underline{a}=R_1\underline{b}=CH_3$, $R_2=CH_3$.

The procedure of Example 13 is followed but procedure 10C replaces procedure 9C.

EXAMPLE 15

Compound I: n=3, R=OH, $R_1\underline{a}=R_1\underline{b}=CH_2CH_3$, $R_2=H$.

The procedure of Example 13 is followed but diethylaminopropylamine is used in place of dimethylaminopropylamine in procedure 1A.

EXAMPLE 16

Compound I: n=3, R=OH, $R_1\underline{a}=R_1\underline{b}=CH_2CH_3$, $R_2=CH_3$.

The procedure of Example 15 is followed but procedure 10C replaces procedure 9C.

Intermediates

Melting points, yields and molecular formulae of starting compounds (III) and intermediates (II) are set forth in Table I.

TABLE I

1-Substituted 4-Nitro-9(10H)-acridinones (III) and 1-Substituted 4-Amino-7-methoxy-9-(10H)-acridinones (II)

| Compd | n | R | $R_1^a = R_1^b$ | mp,°C | yield,% | molecular formula* |
|---|---|---|---|---|---|---|
| (III) | 2 | $OCH_3$ | $CH_3$ | 242-243[d] | 96 | $C_{18}H_{20}N_4O_4$ |
| " | 2 | $OCH_3$ | $CH_2CH_3$ | 178-179[e] | 92 | $C_{20}H_{24}N_4O_4$ |
| " | 3 | $OCH_3$ | $CH_3$ | 165-166 | 94 | $C_{19}H_{22}N_4O_4$ |
| " | 3 | $OCH_3$ | $CH_2CH_3$ | 153-154[f] | 97 | $C_{21}H_{26}N_4O_4$ |
| " | 2 | OH | $CH_3$ | 258-260 | 90 | $C_{17}H_{18}N_4O_4$ |
| " | 2 | OH | $CH_2CH_3$ | 227-229 | 94 | $C_{19}H_{22}N_4O_4$ |
| " | 3 | OH | $CH_3$ | 213-214[g] | 82 | $C_{18}H_{20}N_4O_4$ |
| " | 3 | OH | $CH_2CH_3$ | 208-210 | 86 | $C_{20}H_{24}N_4O_4$ |
| (II) | 2 | $OCH_3$ | $CH_3$ | 240-243 dec. | 79 | $C_{18}H_{22}N_4O_2$.2HCl |
| " | 2 | $OCH_3$ | $CH_2CH_3$ | 227-231 dec. | 74 | $C_{20}H_{26}N_4O_2$.2HCl |
| " | 3 | $OCH_3$ | $CH_3$ | 232-235 dec. | 80 | $C_{19}H_{24}N_4O_2$.2HCl |
| " | 3 | $OCH_3$ | $CH_2CH_3$ | 180-185 dec. | 84 | $C_{21}H_{28}N_4O_2$.3HCl |

*The analyses are within ±0.4% of the theoretical values for C, H and N.

## Final Compounds

Melting points, yields and molecular formulae of compounds of the invention are set forth in Table II, with reference to formula I. It will be appreciated that such compounds can readily be prepared as the free bases or as other acid addition salts and/or hydrates and therefore that the disclosure of Table II is not to be construed as limited by such particular forms of compound. NMR spectra of certain compounds are shown below.

Compound I: R=$OCH_3$, $R_1\underline{a}$=$R_1\underline{b}$=$CH_2CH_3$, $R_2$=H, n=2. (Ex.3)

$^1$H NMR (free base)($Me_2SO-d_6$) δ 9.13(s, 1H,C1-**H**), 8.98(t,1H, ex,-**NH**-$CH_2$-), 8.36(d,1H,J=9.1,C10-**H**), 7.98(d,1H,J=8.9,C3-**H**), 7.79(d,1H,J=3.0,C7-**H**), 7.52(dd,1H,J=9.1,J=3.0,C9-**H**), 6.80(d,1H, J=8.9,C4-**H**), 3.92(s,3H,-$OCH_3$), 3.42(qu*,2H,-NH-$CH_2$-$CH_2$-), 2.73(t, 2H,-$CH_2$-$CH_2$-$NEt_2$), 2.58(qu,4H,-N($CH_2$-$CH_3$)$_2$), 1.02(t,6H,-N($CH_2$-$CH_3$)$_2$).

Compound I: R=$OCH_3$, $R_1\underline{a}$=$R_1\underline{b}$=$CH_2CH_3$, $R_2$=$CH_3$, n=2. (Ex.4)

$^1$H NMR (free base) ($Me_2SO-d_6$) δ 8.98(t,1H,ex,-**NH**-$CH_2$), 8.12(d,1H,J=9.2,C10-**H**), 7.82(d,1H,J=3.2,C7-**H**), 7.80(d,1H,J=8.8, C3-**H**), 7.43(dd,1H,J=9.2,J=3.2,C9-**H**), 6.70(d,1H,J=8.8,C4-**H**), 3.91(s,3H,-$OCH_3$), 3.38(qu*,2H,-NH-$CH_2$-$CH_2$-), 3.00(s,3H,C1-$CH_3$), 2.72(t,2H,-$CH_2$-$CH_2$-$NEt_2$), 2.58(qu,4H,-N($CH_2$-$CH_3$)$_2$), 1.03(t,6H,-N($CH_2$-$CH_3$)$_2$).

Compound I: R=OH, $R_1\underline{a}$=$R_1\underline{b}$=$CH_2CH_3$, $R_2$=H, n=2. (Ex.11)

$^1$H NMR (free base) ($Me_2SO-d_6$) δ 10.00(s,1H,ex,C8-OH), 9.08(s,1H,C1-**H**), 8.99(t,1H,ex,-**NH**-$CH_2$-),

8.26(d,1H,J=8.9,C10-**H**), 7.95(d,1H,J=8.8,C3-**H**), 7.72(d,1H,J=2.8,C7-**H**), 7.33(dd,1H,J=8.9, J=2-8,C9-**H**), 6.77(d,1H,J=8.8,C4-**H**), 3.40(qu*,2H,-NH-$CH_2$-$CH_2$-), 2.70(t,2H,-$CH_2$-$C\mathbf{H}_2$-$NEt_2$), 2.56(qu,4H,-N($C\mathbf{H}_2$-$CH_3$)$_2$), 1.01(t,6H,-N($CH_2$-$C\mathbf{H}_3$)$_2$).

<u>Compound I: R=OH, $R_1^a$=$R_1^b$=$CH_2CH_3$, $R_2$=$CH_3$, n=2. (Ex.12)</u>

$^1$H NMR (free base) ($ME_2SO$-$d_6$) δ 10.00(s,1H,ex,C8-O**H**), 9.02(t,1H,ex,-N**H**-$CH_2$-), 8.11(d,1H,J=9.1,(C10-**H**, 7.83(d,1H,J=8.8, C3-**H**), 7.79(d,1H,J=2.9,C7-**H**), 7.33(dd,1H,J=9.1,J=2.9,C9-**H**), 6.72(d,1H,J=8.8,C4-**H**), 3.38(qu*,2H,-NH-$C\mathbf{H}_2$-$CH_2$-), 3.02(s,3H,C1-$CH_3$), 2.72(t,2H,-$CH_2$-$C\mathbf{H}_2$-$NEt_2$), 2.56(qu,4H,-N($C\mathbf{H}_2$-$CH_3$)$_2$), 1.02(t, 6H,-N($CH_2$-$C\mathbf{H}_3$)$_2$).

<u>Compound I: R=OH,$R_1^a$=$R_1^b$=$CH_2CH_3$,$R_2$=H, n=3. (Ex.15)</u>

$^1$H NMR (free base) ($ME_2SO$-$d_6$) δ 10.02(br s,1H,ex,C8-OH), 9.10(s,1H,C1-**H**), 8.93(t,1H,ex,-N**H**-$CH_2$-), 8.27(d,1H,J=8.9,C10-**H**), 7.97(d,1H,J=8.8,C3-**H**), 7.73(d,1H,J=2.8,C7-**H**), 7.34(dd,1H,J=8.9, J=2.8,C9-**H**), 6.81(d,1H,J=8.8,C4-**H**), 3.42(qu*,2H,-NH-$C\mathbf{H}_2$-$CH_2$-), 2.52(t,2H,-$CH_2$-$CH_2$-$NEt_2$), 2.48(qu,4H,-N($C\mathbf{H}_2$,$CH_3$)$_2$), 1.78(qt,2H,-$CH_2$-$C\mathbf{H}_2$-$CH_2$-), 0.96(t,6H,-N($CH_2$-$C\mathbf{H}_3$)$_2$).

<u>Compound I: R=OH, $R_1^a$=$R_1^b$=$CH_2CH_3$, $R_2$=$CH_3$, n=3. (Ex.16)</u>

$^1$H NMR (free base) ($Me_2SO$-$d_6$) δ 10.0(br s,1H,ex,C8-O**H**), 8.91(t,1H,ex,-N**H**-$CH_2$-), 8.08(d,1H,J=9.1,C10-**H**), 7.80(d,1H,J=8.8, C3-**H**), 7.77(d,1H,J=3.0,C7-**H**), 7.32(dd,1H,J=9.1,J=3.0,C9-**H**), 6.70(d,1H,J=8.8,C4-**H**), 3.38(qu*,2H,-NH-$C\mathbf{H}_2$-$CH_2$-), 3.00(s,3H,C1-$C\mathbf{H}_3$), 2.48(m,6H,-$CH_2$-$CH_2$-N($CH_2$-$CH_3$)$_2$), 1.76(qt,2H,-$CH_2$-$C\mathbf{H}_2$-$CH_2$-).

TABLE II

Formulae, melting points and yields of 8-substituted 5-aminoimidazo[4,5,1-de]acridin-6-ones of formula I

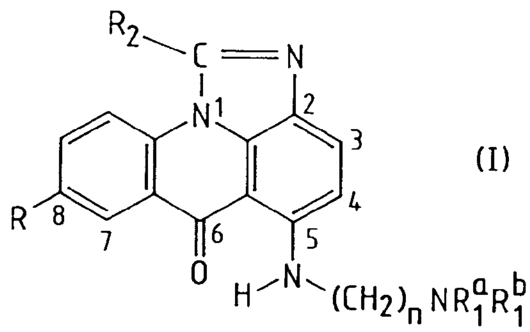

(I)

| EX | n | R | $R_1^a = R_1^b$ | $R_2$ | mp, °C | yield % | formula [c] |
|---|---|---|---|---|---|---|---|
| 1 | 2 | $OCH_3$ | $CH_2CH_3$ | H | 250-254 dec[d] | 68 | $C_{21}H_{24}N_4O_2 . 1.75HCl$ |
| 2 | 2 | $OCH_3$ | $CH_3$ | H | 254-258 dec | 90 | $C_{19}H_{20}N_4O_2 . 1.5HCl\ 0.75H_2O$ |
| 3 | 2 | $OCH_3$ | $CH_3$ | $CH_3$ | 255-259 dec | 82 | $C_{20}H_{22}N_4O_2 . 2\ HCl . H_2O$ |
| 4 | 2 | $OCH_3$ | $CH_2CH_3$ | $CH_3$ | 238-241 dec[e] | 70 | $C_{22}H_{28}N_4O_2 . 2\ HCl$ |
| 5 | 3 | $OCH_3$ | $CH_3$ | H | 237-241 dec | 70 | $C_{20}H_{22}N_4O_2 . 2\ HCl . 0.2H_2O$ |
| 6 | 3 | $OCH_3$ | $CH_3$ | $CH_3$ | 252-256 dec[f] | 68 | $C_{21}H_{24}N_4O_2 . 1.85\ HCl$ |
| 7 | 3 | $OCH_3$ | $CH_2CH_3$ | H | 246-250 dec | 72 | $C_{22}H_{26}N_4O_2 . 1.5\ HCl$ |
| 8 | 3 | $OCH_3$ | $CH_2CH_3$ | $CH_3$ | 203-208 dec | 64 | $C_{23}H_{28}N_4O_2 . 2\ HCl . H_2O$ |
| 9 | 2 | OH | $CH_3$ | H | 260-264 dec | 77 | $C_{18}H_{18}N_4O_2 . 2\ HCl . H_2O$ |
| 10 | 2 | OH | $CH_3$ | $CH_3$ | 268-273 dec | 68 | $C_{19}H_{20}N_4O_2 . 2\ HCl . 2H_2O$ |
| 11 | 2 | OH | $CH_2CH_3$ | H | 250-255 dec[g] | 72 | $C_{20}H_{22}N_4O_2 . 2\ HCl . H_2O$ |
| 12 | 2 | OH | $CH_2CH_3$ | $CH_3$ | 260-265 dec[h] | 78 | $C_{21}H_{24}N_4O_2 . 1.5HCl\ 0.5H_2O$ |
| 13 | 3 | OH | $CH_3$ | H | 247-251 dec | 70 | $C_{19}H_{20}N_4O_2 . 2\ HCl$ |
| 14 | 3 | OH | $CH_3$ | $CH_3$ | 268-271 dec[i] | 69 | $C_{20}H_{22}N_4O_2 . 2\ HCl . 0.5H_2O$ |
| 15 | 3 | OH | $CH_2CH_3$ | H | 269-272 dec[j] | 70 | $C_{21}H_{21}N_4O_2 . 2\ HCl . H_2O$ |
| 16 | 3 | OH | $CH_2CH_3$ | $CH_3$ | 238-242 dec[k] | 66 | $C_{22}H_{26}N_4O_2 . 2\ HCl . H_2O$ |

(c) Microanalyses are within ±0.4% of the theoretical values for C, H and N. (d) Free base mp 191-193 °C. (e) Free base mp 156-158 °C. (f) Free base mp 156-158 °C. (g) Free base mp 239-242 °C. (h) Free base mp 255-258 °C. (i) Free base mp 242-245 °C. (j) Free base mp 222-225 °C. (k) Free base mp 240-245 °C.

## Biological Tests

### In Vitro Cytoxicity Evaluation

The mouse L1210 leukaemia cells (RPMI, USA) are grown in RPMI 1640 medium supplemented with 5% fetal calf serum and penicillin (1,000,000 units /litre) plus streptomycin (100 mg/litre) in controlled air-5% $CO_2$ humidified atmosphere at 37°C. L1210 mouse leukemia cells are seeded at a density of $5x10^4$ cells/ml. The tested compounds, dissolved in 50% ethanol, are added, at four different concentrations, to the cell suspensions. The cytotoxic activity ($IC_{50}$ value) of the tested compounds is defined as their concentrations causing 50% growth inhibition after 48h, measured by cell protein contents and is determined from dose-response curves by the method of J. Konopa, A. Matuszkiewicz, M. Hrabowska, K. Onoszko, Arzneim.-Forsch. 1974, 24, (1971).

### In Vivo Antileukaemic Evaluation

$BDF_l$ mice are injected ip with $10^6$ P388 lymphotic leukemia cells on day O and treated ip on days 1-5 in accordance with the protocols described by the National Cancer Institute :
R.I. Geran, et al., Cancer Chemotherapy Reports, Part 3, 3(2), 1-103 (1972) [ISSN=0069-0139]. The mean survival time (MST) for each treatment group (eight mice) is calculated and the percent of T/C was determined by using the following formula:

$$\%T/C = [(MST\ treated)/(MST\ control)]x100.$$

Results of the Cytotoxicity Evaluation and Antileukaemic Evaluation are set forth in Table III:

TABLE III

Formulae and activities against Murine L1210 *in vitro* and P388 leukemia *in vivo* of 8-substituted 5-aminoimidazo[4,5,1- de]acridin-6-ones of formula I

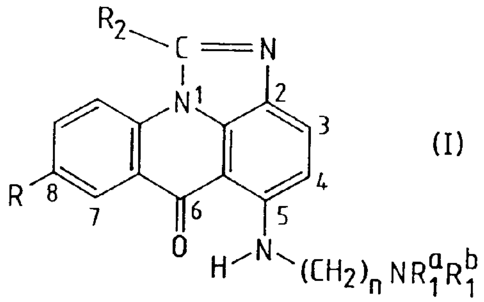

(I)

| EX | n | R | $R_1^a = R_1^b$ | $R_2$ | L1210 leukaemia *in vitro* $IC_{50}$ (µg/ml) | (µM) | P 388 leukaemia *in vivo* optimal dose (mg./kg.) | %* TC |
|---|---|---|---|---|---|---|---|---|
| 1 | 2 | $OCH_3$ | $CH_2CH_3$ | H | 0.78(±0.02) | 1.8 | 100 | 183,209 |
| 2 | 2 | $OCH_3$ | $CH_3$ | H | 0.65(±0.07) | 1.6 | 100 | 177,136 |
| 3 | 2 | $OCH_3$ | $CH_3$ | $CH_3$ | 0.34(±0.09) | 0.77 | 150 | 127 |
| 4 | 2 | $OCH_3$ | $CH_2CH_3$ | $CH_3$ | 0.70(±0.40) | 1.55 | 150 | 136,120 |
| 5 | 3 | $OCH_3$ | $CH_3$ | H | 3.50(±0.75) | 8.25 | 150 | 164 |
| 6 | 3 | $OCH_3$ | $CH_3$ | $CH_3$ | 1.40(±0.60) | 3.2 | 150 | 136 |
| 7 | 3 | $OCH_3$ | $CH_2CH_3$ | H | 1.10(±0.32) | 2.5 | 150 | 142 |
| 8 | 3 | $OCH_3$ | $CH_2CH_3$ | $CH_3$ | 0.70(±0.17) | 1.4 | 150 | 108 |
| 9 | 2 | OH | $CH_3$ | H | 0.02(±0.01) | 0.048 | 12.5 | 210,210 |
| 10 | 2 | OH | $CH_3$ | $CH_3$ | 0.06(±0.03) | 0.135 | 12.5 | 200,250 |
| 11 | 2 | OH | $CH_2CH_3$ | H | 0.013(±0.008) | 0.031 | 5 | 211,175 |
| 12 | 2 | OH | $CH_2CH_3$ | $CH_3$ | 0.11(±0.08) | 0.25 | 75 | 280,290 |
| 13 | 3 | OH | $CH_3$ | H | 0.014(±0.005) | 0.034 | 5 | 183 |
| 14 | 3 | OH | $CH_3$ | $CH_3$ | 0.10(±0.07) | 0.23 | 100 | 255 |
| 15 | 3 | OH | $CH_2CH_3$ | H | 0.08(±0.05) | 0.18 | 25 | 309,230 |
| 16 | 3 | OH | $CH_2CH_3$ | $CH_3$ | 0.40(±0.21) | 0.89 | 25 | 150,155 |

* When two values are given, the second one represents the result obtained during repeated independent multidose assay.

## Free-radical testing

Many anti-cancer compounds exhibit cardiotoxic side effects which are usually ascribed to the liberation

of free radicals. Accordingly four representative compounds of the invention, those of Examples 1, 11, 12 and 15, were tested in rat liver microsomes, in comparison with doxorubicin. Whereas 500μM of doxorubicin in NADPH-supplemented rat liver microsomes, under nitrogen purging, gave an electron spin resonance (ESR) signal indicative of the presence of doxorubicin semiquinone free radical, the same molar concentration (500μM) of the four compounds of the invention gave no ESR spectrum over a three hour period.

## Claims

1. Compounds of formula I:

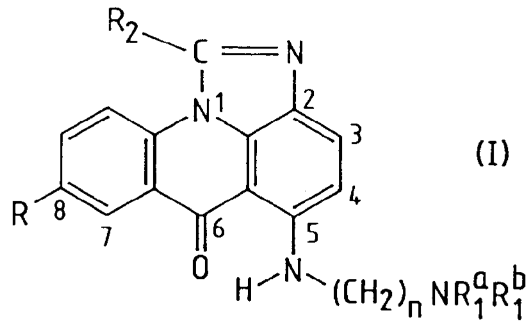

(I)

in which:
R represents: -OH or -OR′, wherein R′ represents $C_1$-$C_6$ alkyl,
$R_1^a$ and $R_1^b$, which may be identical or different, represent hydrogen or $C_1$-$C_6$ alkyl, unsubstituted or substituted by a hydroxyl, an amino, a N′-alkylamino or a N′,N′-dialkylamino group, such N′-alkyl groups containing 1-4 carbon atoms,
n is 2-5 and
$R_2$ represents hydrogen, or straight chain $C_{1-4}$ alkyl, in the form of a free base or a pharmaceutically acceptable acid addition salt, or an N-oxide thereof.

2. Compounds according to Claim 1, in which $R_1^a$ and $R_1^b$ are identical.

3. Compounds according to claim 1 or 2, in which $R_1^a$ or $R_1^b$ or both represent a $C_1$-$C_3$ alkyl group.

4. Compounds according to claim 1, 2 or 3, in which n is 2 or 3.

5. Compounds according to claim 1, in which R = -OH, $R_1^a$ = $R_1^b$ = methyl or ethyl, $R_2$ = hydrogen or methyl and n is 2 or 3.

6. A process for the production of a compound of formula I claimed in claim 1, wherein a compound of formula II, optionally in the form of an acid addition salt thereof,

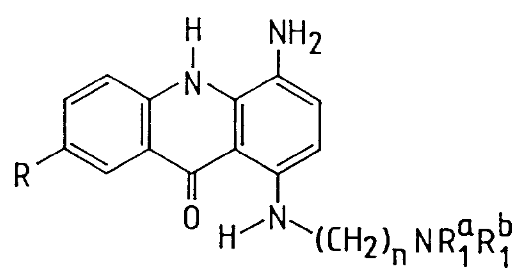

(II)

is treated with formic acid or an N,N-dialkylamide of formula $R_2CON(CH_3)_2$, $R_2$ representing straight chain $C_{1-4}$ alkyl, and, if necessary, an acid addition salt is converted to the free base or the free base to a pharmaceutically acceptable acid addition salt or to an N-oxide.

7. Compounds according to claim 1 for use in therapy.

8. The use of a compound according to claim 1 for the manufacture of a medicament useful in antineoplastic treatment or prophylaxis.

9. A pharmaceutical composition which comprises a compound according to claim 1, 2, 3, 4 or 5, in association with a pharmaceutically acceptable diluent or carrier therefor.

10. Each of the individual compounds according to claim 1 hereinbefore set forth, in the form of free bases or any of their pharmaceutically acceptable acid addition salts.

European Patent Office

# EUROPEAN SEARCH REPORT

Application Number

EP 92 30 1732

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF MEDICINAL CHEMISTRY. vol. 33, no. 1, January 1990, WASHINGTON US pages 49 - 52; W. M. CHOLODY ET AL.: '5-[(Aminoalkyl)amino]imidazo[4,5,1-de]acridin-6-ones as a Novel Class of Antineoplastic Agents. Synthesis and Biological Activity"' * the whole document * | 1,8 | C07D471/06<br>A61K31/435<br>// (C07D471/06, 235:00, 221:00) |
| Y | * the whole document * | 1-10 | |
| Y | JOURNAL OF MEDICINAL CHEMISTRY. vol. 33, no. 10, October 1990, WASHINGTON US pages 2852 - 2856; W. M. CHOLODY ET AL.: '8-Substituted 5-[(Aminoalkyl)amino]-6H-v-triazolo[4,5,1-de]acridin-6-ones as Potential Antineoplastic Agents. Synthesis and Biological Activity' * the whole document; see especially page 2854, left column, line 3 - 17 * | 1-10 | |
| P,X | JOURNAL OF MEDICINAL CHEMISTRY. vol. 35, no. 1, 24 January 1992, WASHINGTON US pages 378 - 382; W. M. CHOLODY ET AL.: 'Chromophore-Modified Antineoplastic Imidazoacridinones. Synthesis and Activity against Murine Leukemias' * the whole document * | 1-10 | TECHNICAL FIELDS SEARCHED (Int. Cl.5)<br>C07D |

The present search report has been drawn up for all claims

| Place of search | Date of completion of the search | Examiner |
|---|---|---|
| THE HAGUE | 28 APRIL 1992 | SEUFERT G.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)